# FASCICULE DE BREVET EUROPEEN

(11) **EP 1 358 481 B1**
(45) Date de publication et mention de la délivrance du brevet: **30.06.2004**
(21) Numéro de dépôt: 01919525.4
(22) Date de dépôt: 16.03.2001
(51) Int. Cl.: G01N 33/543, C12Q 1/68, A61K 49/00, A61B 1/00

(54) **DISPOSITIF D'ANALYSE ET/OU DE TRAITEMENT AVEC UNE TIGE SOUPLE**
VORRICHTUNG ZUR ANALYSE UND/ODER ZUR BEHANDLUNG MIT EINEM FLEXIBLEN SCHAFT
DEVICE FOR IN SITU ANALYSIS AND/OR TREATMENT CONSISTING OF A FLEXIBLE ROD AND A MICRO-SYSTEM FIXED AT ONE END OF SAID FLEXIBLE ROD

(30) Priorité: 17.03.2000 FR 0003474; 08.11.2000 US 246571 P
(43) Date de publication de la demande: 05.11.2003
(73) Titulaire: Pompidou, Alain, 75004 Paris (FR); Benhamou, Albert-Claude, 75005 Paris (FR)
(72) Inventeur: Pompidou, Alain, 75004 Paris (FR); Benhamou, Albert-Claude, 75005 Paris (FR)
(74) Mandataire: Breese, Pierre
(86) Numéro de dépôt international: PCT/FR2001/000803
(87) Numéro de publication internationale: WO 2001/069257

(56) Documents cités:
- WO-A-00/63437
- WO-A-98/50782
- US-A- 5 001 051
- US-A- 5 804 453
- US-A- 5 837 196
- US-A- 5 938 595
- DATABASE WPI Section Ch, Week 199410 Derwent Publications Ltd., London, GB; Class B04, AN 1994-077443 XP002156073 SAKAI YASUSHI ET AL.: "IMMUNOLOGICAL EXAMINATION METHOD FOR CANCER AND ENDOSCOPE FOR IT" & JP 06 027110 A (IBIDEN CO LTD), 4 février 1994 (1994-02-04)

## Description

La présente invention concerne un dispositif permettant de réaliser à distance une investigation et/ou un traitement *in situ* au niveau d'un substrat par exemple de tissus ou d'organes, constitué d'une tige souple à une extrémité de laquelle est fixé un micro-système d'investigation et/ou de traitement.

Il est décrit dans l'art antérieur des microsystèmes d'investigation mettant en oeuvre un arrangement de molécules biologiques disposées en des positions déterminées d'une surface. Ces systèmes connus sous la dénomination de " biopuces " ou de puces à ADN sont utiles pour l'investigation de séquences polynucléotidiques ou d'acides aminés. Des exemple de tels systèmes sont décrits par exemple dans les demandes de brevet européen publiées sous le No. 619 321, No. 373 203, No. 691 978. D'autres microsystèmes d'investigation sont par exemple les tests de microanalyse mettant en oeuvre des réactions du type ligand/récepteur ou des microimmunoanalyse mettant en oeuvre des réactions du type antigène/anticorps.

On connaît également dans l'art antérieur des dispositifs d'investigation *in vivo* des organes ou tissus du type d'un cathéter constitué d'un tube flexible inséré dans les vaisseaux ou par les voies naturelles, associés par exemple à un laser, une fibre optique, une sonde ou un capteur. Ces dispositifs peuvent également permettre l'administration de substances actives comme des médicaments ou des agents de diagnostic, comme par exemple le dispositif décrit dans le brevet américain US 5,938,595.

Ce document concerne un dispositif d'analyse et de traitement comprenant une fibre optique dont l'une des extrémités est recouverte d'une enveloppe comprenant des anticorps taggés avec une molécule fluorescente. Dans le cas où ce dispositif est utilisé à des fins de traitement (par exemple de maladies vasculaires), ladite fibre optique est intégrée dans un cathéter qui est amené à travers le système vasculaire jusqu'au site d'occlusion et des agents thrombolytiques peuvent être relargués directement dans le caillot. Ce système peut être associé à un autre dispositif de traitement, tel que laser, utilisé pour mieux détruire le caillot

Le brevet américain US 5,837,196 décrit un biocapteur constitué par des fibres optiques qui peut éventuellement être utilisé *in situ* chez l'homme et chez l'animal à des fins de détection d'une multitude d'analytes. Ce document décrit une méthode de fixation d'une substance biologique à une surface solide au moyen d'une matrice polymère. Ainsi, ce biocapteur est préparé par fixation à l'extrémité de chacune des fibres optiques du faisceau une substance biologique qui permettra la détection des analytes cibles.

Les inventeurs ont maintenant conçu un dispositif d'analyse et/ou de traitement *in situ* manoeuvrable à distance associant les deux technologies ci-dessus. Il apparaît en effet utile de disposer de nouveaux moyens d'investigation et/ou de traitement *in situ* au niveau d'un substrat d'un organisme *in vivo* ou *in vitro* le moins traumatisant possible particulièrement dans le cas d'un patient humain et qui permette d'accéder (i) à des informations utiles tant en matière de diagnostic que de criblage par exemple pour des indications thérapeutiques, ou (ii) à des nouveaux modes d'administration d'agents actifs drogues, directement au niveau d'un substrat constitué par exemple de cellules cibles.

Ce but est atteint selon la présente invention grâce à un dispositif pour l'analyse et/ou le traitement *in situ* dans lequel un système d'investigation et/ou de traitement coulisse dans un instrument médical, caractérisé en ce qu'il comprend (i) un micro-système d'investigation d'un substrat autre que par analyse d'un signal fluorescent, et/ou de délivrance d'agents actifs au niveau d'un substrat, (ii) une tige souple à une extrémité de laquelle est fixé le micro-système et dont l'autre extrémité est destinée à la manoeuvre dudit micro-système, (iii) un instrument médical possédant une lumière interne dans laquelle ladite tige souple peut coulisser, (iv) un système de protection du micro-système amovible au niveau du substrat, et (v) au niveau dudit micro-système, un système de dilacération de tissu ou de cellule.

Avantageusement, ledit microsystème et ladite tige souple sont maintenus solidaires *in situ.* Ainsi, selon l'invention, ledit microsystème et ladite tige souple demeurent reliés l'un à l'autre lors de leur utilisation *in situ* et ne sont désolidarisés qu'avant ou après l'intervention in situ de façon à ce que le microsystème ne constitue pas un système implantable ou largable in situ.

Dans une première forme de réalisation, le micro-système est du type comprenant un support à la surface duquel sont ménagées des régions prédéfinies contenant chacune différentes substances chimiques ou biologiques d'investigation ou de traitement du substrat au contact duquel est amené le micro-système grâce à la tige souple. Ladite surface comprend au moins deux, de préférence plus de 100 et tout préférentiellement plus de 1000 régions prédéfinies sur une surface de l'ordre de quelques cm² de préférence de l'ordre de 1 cm² ou moins. Chaque zone prédéfinie comporte une substance chimique ou biologique différente, mais le procédé selon l'invention admet également que plusieurs voire toutes lesdites régions prédéfinies comportent la même substance chimique ou biologique par exemple dans le cas de la même analyse ou de la délivrance de la même substance active dans le temps.

Le support peut comprendre plusieurs face dont l'une au moins est constituée d'une surface active. Celle-ci est le siège d'un ou plusieurs agents de liaison biologiques. Au milieu de ladite surface active sont ménagées des régions prédéfinies.

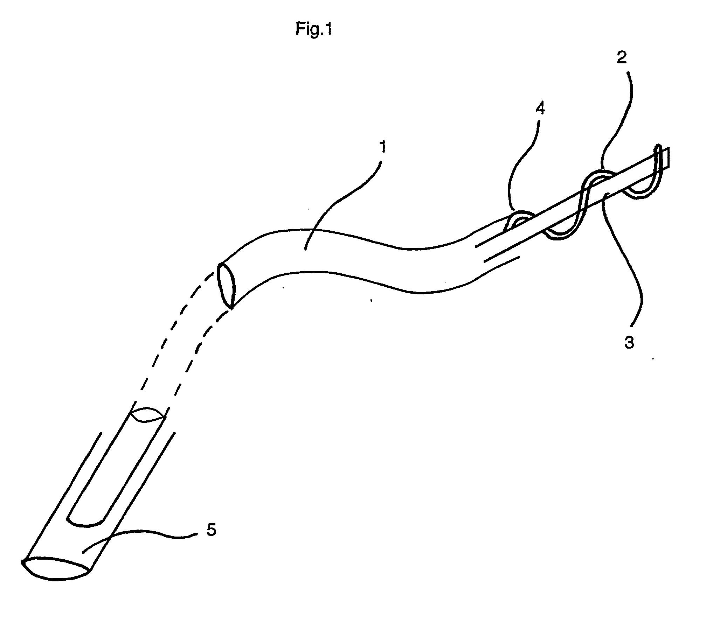

Cette surface de l'ordre d'un cm² peut être plane et est située dans un seul plan, elle peut également être conformée en ruban et s'enrouler en spirale autour d'un support rigide qui prolonge la tige souple et qui présente alors le microsystème de même nature que le précédent, mais de conformation plus élaborée. La figure 1 représente un exemple de réalisation d'un dispositif selon l'invention constitué d'une tige souple (1), comme par exemple un cathéter deformable, à l'extrémité de laquelle est insérée, dans la lumière de la tige souple le microsystème (2) constitué d'un support (3) sur lequel est enroulé un ruban (4) ou son fixés des anticorps spécifiques d'un antigène présent au niveau du substat analysé. Le support (3) est rigide alors que la tige souple (1) est relativement deformable. La tige souple (1) peut être associée à un système d'exploration endovasculaire (5) comme par exemple un endoscope.

Le dispositif de l'invention peut être associé à un système d'exploration endocavitaire ou endovasculaire permettant d'amener le microsystème au contact ou à proximité du substrat cible.

Dans une seconde forme de réalisation, le dispositif de l'invention peut être composé d'une tige souple, éventuellement associé à un système d'exploration endocavitaire ou endovasculaire. Celle-ci est terminée par un microsystème constitué d'un segment articulé composé d'une alternance de substances rigides (type billes de platine) et de gel plus ou moins hydrophile (hydrogel) sur lequel sont fixés les groupements, molécules ou substances réactives. La couche active entourant le support rigide peut-être un ruban plan ou une corde ronde sur lesquels les réactifs sont déposés et fixés. Il peut s'agir de ligands et notamment d'antigènes ou d'anticorps, mais également de nucléotides. Ainsi, dans ce mode de réalisation, le microsystème est constitué d'un support se présentant sous la forme d'un ruban enroulé autour d'une estrémité de la tige souple. Un exemple de cette forme de réalisation est représentée à la figure 1 des dessins en annexe.

Comme indiqué précédemment, chaque zone prédéfinie peut comporter la même substance active chimique ou biologique, par exemple dans le cas de la même analyse ou de la délivrance de la même substance active reproduits dans le temps. Chaque zone prédéfinie peut aussi comporter une substance chimique ou biologique différente, et le micro-système supportant alors une pluralité de substances chimiques ou biologiques différentes est destiné à effectuer une analyse multiple.

Le dispositif selon l'invention est utile dans :
- le domaine thérapeutique, pour délivrer in situ des agents actifs au niveau d'un substrat, et
- le domaine du diagnostic pour l'analyse in situ d'un substrat,

En particulier, le dispositif de l'invention est utile pour des applications thérapeutiques, menées dans le cadre de la thérapie guidée, telle qu'elle est proposée pour la thérapie génique intracardiaque visant à mener *in situ* des cellules myoïdes autologues après avoir évalué à partir de la sonde endocavitaire l'état tissulaire cellulaire de la zone myocardique à traiter. Ceci à l'aide de différents systèmes et plus particulièrement des microsystèmes de diagnostique ou de suivi thérapeutique de l'invention.

Dans le cadre des applications thérapeutique, les substances biologiques et ou chimiques disposées au niveau de chaque région prédéfinie de la surface du microsystème sont des agents actifs, notamment des substances thérapeutiques. Les agents actifs peuvent être toute substance utile dans le traitement des maladies nécessitant une intervention au niveau de cellules ou de tissus cibles, comme des cellules cancéreuses, des foyers infectieux, etc.

Dans le cadre des applications diagnostiques, les substances biologiques et ou chimiques disposées au niveau de chaque région prédéfinie de la surface du microsystème sont des substances permettant de détecter des analytes spécifiques du substrat ou est amené le microsystème, ou encore des substances radiomarquées permettant la visualisation à distance de l'organe ciblé, par toute technique connue d'imagerie médicale.

Lesdites substances peuvent être fixées, au niveau de chaque région prédéfinie du support soit de manière réversible, pour éventuellement être relargués par le microsystème pour qu'ils exercent leur fonction « in vivo », après retrait dudit microsystème, soit de manière irréversible, à ladite surface. La fixation peut être réalisée par simple adsorption ou par l'intermédiaire d'un produit de couplage.

Les agents actifs sont libérés au niveau du substrat :
- par simple contact avec celui-ci, auquel cas, ils sont protégés physiquement comme décrit plus loin, jusqu'à ce que le micro-système soit placé au niveau dudit substrat, ou
- grâce à un système complémentaire de libération au niveau du microsystème.

Une forme de mise en oeuvre préférée de l'application thérapeutique du dispositif de l'invention consiste à utiliser des substances actives qui sont des acides nucléiques, comme des ADN nus, des oligonucléotides antisens, fixés à la surface du support du micro-système par hybridation. Mais les substances actives peuvent être aussi des protéines ou des anticorps fixés sur chaque région prédéfinie grâce à une liaison immunologique. A titre d'exemple d'un micro-système entrant dans la constitution d'un dispositif de traitement selon l'invention, on peut citer les puces bioniques qui contrôlent électriquement l'activité des cellules de façon à libérer des agents thérapeutiquement actifs.

Pour les applications de diagnostic et plus largement d'analyse d'un substrat, le micro-système du dispositif de l'invention est un micro-système d'investigation.

Selon une forme de réalisation toute préférée du dispositif selon l'invention, les substances chimiques ou biologiques sont capables de réagir avec des substances correspondantes éventuellement présentes au niveau du substrat où est amené le micro-système d'investigation grâce à la tige souple.

Ainsi, le micro-système est de préférence un micro-système récepteur/ligand mettant en oeuvre des couples de substances chimiques ou biologiques dont l'un ou l'autre ou, notamment pour des applications thérapeutiques, les deux membres du couple sont fixés sur chaque région prédéfinie.

Avantageusement, le micro-système selon l'invention met en oeuvre des substances biologiques qui sont des séquences polynucléotidique ou d'acides aminés. L'invention envisage comme substances chimiques ou biologiques présents dans chaque région prédéfinie, des substances chimiques ou biologiques constituant:
- un arrangement de séquences polynucléotidique capable de s'hybrider avec des acides nucléiques présents au niveau du substrat constituant notamment le site d'investigation, ou
- un arrangement de peptides, polypeptides ou protéines capables de réagir avec un récepteur correspondant ou immunologiquement avec des anticorps ou des antigènes présents au niveau du substrat constituant notamment le site d'investigation.

Mais, le micro-système d'investigation peut aussi mettre en oeuvre des substances chimiques capables de réagir selon des réactions chimiques variées avec des substances chimiques correspondantes présentes au niveau du substrat analysé. Ainsi, le micro-système d'investigation selon l'invention permet l'analyse simultanée de plusieurs facteurs physiologiques in situ.

Compte tenu de la diversité des types de réactions biologiques ou chimiques susceptibles d'être mises en oeuvre au niveau du micro-système d'investigation, le dispositif de l'invention permet l'identification de gènes ou de leurs composants d'ADN, d'ARN, de séquences nucléotidiques pertinentes ou de leurs produits protéiques spécifiques, mais aussi d'agonistes et de leurs récepteurs. I1 permet également *in vitro* et *in vivo* l'identification de virus, de bactéries, de parasites ou de leurs composants spécifiques, ainsi que des agents pathogènes de type prion.

Le dispositif de l'invention peut ainsi être utilisé :
- à des fins d'analyse des divers types de génomes et notamment à leur séquençage,
- à l'identification de cellules, de tissus et d'organes ou de différents types de récepteurs spécifiques normaux ou pathologiques,
- à des fins de diagnostic, comme par exemple grâce à l'identification de gènes, de leur composant ou de leur produit,
- à des fins de criblage de molécules ou de substances chimiques ou biologiques à caractéristiques thérapeutiques connues ou potentielles,
- au suivi de l'activité d'agents thérapeutiques nouveaux ou déjà connus (possibilité d'examens orientés étagés et répétés).

La liste ci-dessus n'est pas exhaustive et l'homme du métier est capable à l'aide des présentes indications d'étendre la mise en oeuvre du dispositif de l'invention à tout autre type d'analyse, tels que ceux qui font intervenir la liaison antigène-anticorps ou les systèmes ligand dans la mesure ou au moins pour partie les liaisons intervenant in vivo sont détectables à l'aide des microsystèmes décrits, même si des liaisons supplémentaires sont réalisées ultérieurement pour la révélation *ex vivo* ou *in vitro* d'une réaction qui s'est produit *in vivo* et *in situ*.

Le dispositif de l'invention offre tout particulièrement une amélioration significative aux méthodes de diagnostic, d'indication thérapeutique, de traitement et de suivi thérapeutique ainsi que de criblage des médicaments qui sont issus de la génomique et de la protéinomique (ou protéomique).

Le dispositif de l'invention est remarquable en ce qu'il est non-invasif ou micro-invasif non destructif et qu'il permet l'identification à distance de toutes substances chimiques ou biologiques capables de réagir plus ou moins spécifiquement avec les substances actives fixées sur micro-système d'investigation. Il peut s'agir notamment de molécules ou de substances présentes exclusivement *in situ* dans l'organe, le tissu, ou au niveau des cellules (fusion intracellulaire). Cette présence exclusive est liée au fait que ces molécules ou substances sont rapidement métabolisées en dehors du tissu étudié dès qu'elles sortent de leur environnement local. Ceci rend compte de l'intérêt d'opérer *in situ* à l'aide du microsystème décrit.

Il est donc adapté à une analyse ou un traitement :
- *in vitro* : sur cultures de cellules, de tissus ou d'organes naturels ou modifiés génétiquement provenant de prélèvements effectués sur des êtres vivants.
- *in vivo* : dans des conditions invasives ou micro-invasives. Il permet le guidage, l'orientation et le positionnement du microsystème destinée notamment à l'hybridation moléculaire *in situ* au niveau de cellules, de tissus ou d'organes cibles.

Le dispositif de l'invention est en outre particulièrement remarquable car il permet d'atteindre, pour l'analyse ou le traitement, des sites non accessibles ou difficilement accessibles par les techniques conventionnelles.

La tige souple permet d'amener puis de positionner le micro-système au contact du substrat à analyser, qu'il s'agisse d'un matériel biologique vivant ou ayant vécu (frais, fixé, congelé, momifié ou fossilisé), constitué de cellules identiques ou comparables, de tissus mono ou pluricellulaires, d'organes, directement ou après microeffraction du mésothelium ou de l'épithélium de revêtement et de la capsule conjonctive ainsi que des endothéliums vasculaires en cas d'exploration endovasculaire. La tige souple assure la solidité et la cohésion du dispositif et assure sa flexibilité et la transmissibilité des mouvements imprimés à distance manuellement ou de façon robotisée. Bien entendu le dispositif doit être biocompatible et avantageusement stérile, tout au moins en sa partie terminale en contact avec le substrat à traiter ou analyser.

Le dispositif de l'invention comprend donc, outre le micro-système où sont réalisées les réactions chimiques biologiques, une tige qui permet le contrôle manuel ou robotisé du dispositif.

La tige souple de manoeuvre du dispositif permet d'assurer au moins les deux fonctions suivantes :
- Le guidage du micro-système jusqu'au niveau du substrat analysé ou traité.
- L'orientation et le positionnement du microsystème au niveau du substrat analysé ou traité, y compris l'embouchage dans un cathéter souple qui sera lui-même guidé au départ par un système d'exploration endocavitaire
ou endovasculaire. Dans certains cas le microsystème peut être embouché directement à l'extrémité creuse du système d'exploration endocavitaire ou endovasculaire.

La tige souple est avantageusement conçue pour coopérer avec différents instruments médicaux utilisés à des fins diagnostiques, thérapeutiques ou expérimentales, il s'agit de dispositifs :
- non invasifs, destinés à l'exploration de cavités naturelles en contact avec le milieu extérieur, comme des instruments d'exploration ORL, bronchopulmonaire, digestive, urologique ou gynécologique ;
- microinvasifs, en vue de l'exploration des cavités naturelles sans contact direct avec le milieu extérieur, il s'agit alors des instruments utilisés en arthroscopie, coloscopie, etc., ou de système d'exploration endovasculaire, ou encore d'outil de biopsie de tissus ou d'organes superficiels, notamment par voie transcutanée, comme des systèmes d'effraction parenchymateuse, tels que des aiguilles, des pointes acérés, des dispositifs coupants, etc.

La tige souple est ainsi associée à l'un de ces instruments médicaux. Selon une forme préférée de réalisation, la tige souple est micro-adaptée de façon à être introduite dans les instruments ci-dessus possédant une lumière interne. La tige souple peut alors coulisser dans cette lumière interne à l'aide éventuellement d'une rainure de guidage ménagée spécialement à cet effet et ainsi permettre la manoeuvre du dispositif de l'invention in vivo au cours de différentes étapes d'une exploration à visée diagnostic ou thérapeutique.

Mais la tige souple peut aussi consister en ces différents instruments médicaux eux-mêmes, qui sont alors utilisés comme tige de manoeuvre du micro-système d'investigation ou de traitement.

Le micro-système est fixé à l'une des extrémités de la tige souple, soit à l'extérieur de celle-ci, soit inséré dans la lumière de ladite tige souple, par tout moyen de liaison. Ce moyen de liaison peut être un système de pivot permettant l'orientation et le positionnement à partir de la tige souple dans l'espace et à distance du micro-système. Le moyen de liaison peut être constitué par un matériau déformable contrôlé électroniquement et à distance. Il peut s'agir aussi d'une colle biologique et notamment d'un type de colle utilisée pour la réparation de tissus osseux.

Dans tous les cas, le moyen de liaison doit répondre aux critères suivants : adhérence, solidité et flexibilité (résistance aux manipulations et aux mises en tension liées aux mouvements imprimés à distance), biocompatibilité et stérilité.

Le dispositif de l'invention peut être réalisé à partir d'un moule de configuration adaptative dans lequel un ou plusieurs matériaux homogènes ou à composants électroniques sont coulés afin de prendre la forme souhaitée. Le dispositif destiné à réaliser le système pivot et éventuellement un cache temporaire peut être coulé à un endroit choisi du moule soit sous forme de dispositif intermédiaire, soit sous forme de matériau adapté afin de permettre la réalisation de l'ensemble du dispositif décrit plus haut. Il gagne ainsi en efficacité tout en gardant sa cohérence fonctionnelle.

Le moyen de liaison peut être actionnable à distance pour libérer le micro-système in situ. Ainsi, le dispositif de l'invention comprend les deux modes de réalisations décrits ci-dessous.

Selon un premier mode de réalisation du dispositif de l'invention, la tige de manoeuvre permet d'introduire, de positionner puis d'extraire le microsystème après que les réactions chimiques biologiques recherchées se soient produites au niveau du substrat.

Afin d'améliorer le contact entre le microsystème disposé *in situ* et le substrat, un mouvement de rotation ou de va-et-vient peut-être imprimé localement au microsystème à l'aide de la tige souple de guidage, que celle-ci soit pleine (le microsystème est alors fixé à son extrémité par un système adéquat, éventuellement une rotule robotisée). ou que celle-ci soit creuse, tubulaire, afin de recevoir la base du microsystème qui s'y trouve donc partiellement enfoncé. L'objectif est de dilacérer le tissu, voire de lyser les cellules par l'apport de substances réactives adéquates, amenées par le cathéter jusqu'au microsystème.

Ces opérations sont contrôlées soit manuellement soit par l'intermédiaire d'une assistance robotique. Pour des applications d'analyse d'un substrat, une fois récupéré le micro-système est analysé au laboratoire par les techniques classiques de révélation des réactions.chimiques notamment du type récepteur/ligand qui se sont produites, éventuellement après des étapes préparatives comme une réaction de polymérisation en chaîne. Dans ce mode de réalisation, le micro-système est libéré de la tige souple après extraction et le moyen de liaison ne nécessite pas d'être actionnable à distance. Le moyen de liaison doit cependant assurer une cohésion et une flexibilité suffisante pour permettre le contrôle à distance en vue du guidage, de l'orientation, et du positionnement et éventuellement l'extraction du microsystème *in situ.*

Selon un second mode de réalisation du dispositif de l'invention, le moyen de liaison entre la tige souple et le micro-système est actionnable à distance de façon à positionner le dispositif au niveau du site à analyser. Le transport et positionnement du micro-système peut être réalisé à l'aide d'un ballonnet compatible stérile. Dans ce mode de réalisation, le micro-système peut être analysé à distance grâce à des systèmes de capteurs permettant l'analyse des réactions chimiques ou biologiques qui se sont éventuellement produites au niveau du microsystème d'investigation. Tout microprocesseur ou tout dispositif susceptible d'augmenter la sensibilité, l'efficacité et donc la performance du micro-système ou le contrôle à distance de la mise en place (guidage, orientation et positionnement) de la microbiopuce entre dans le cadre et peut être mis en oeuvre dans le cadre du dispositif selon l'invention.

Le dispositif de l'invention peut comprendre un système de protection du micro-système d'investigation qui est déprotégé une fois amené et positionné sur le site à analyser. Ce système de protection, par exemple un rideau amovible, un filet déformable peut couvrir tout le microsystème ou seulement sa surface active. Ce système de protection peut être situé au niveau du moyen de liaison ou placé au niveau du micro-système lui-même.

Le dispositif de l'invention peut comprendre, au niveau de l'extrémité où est fixé le micro-système, tout système d'aide au fonctionnement du micro-système, comme un système de chauffage et/ou de refroidissement, de libération de substance biologique ou chimique comme des tampons, des réactifs de révélation, tout produit de modification biologique ou toutes molécules de l'environnement cellulaire.

Il peut être complété par tout système susceptible de dilacérer le tissu examiné ou par un système apportant des substances susceptibles de lyser sur place les cellules afin d'avoir accès au contenu de celles-ci, tout en restant in situ le temps nécessaire à une réaction de fixation optimale des molécules du substrat sur celles de la couche réactive.

Le dispositif de l'invention peut être associé à tout dispositif permettant à distance :
- la surveillance par des récepteurs sensoriels (tactiles, optiques, physico-chimiques et notamment électroniques ou informatiques numérisés) ou pour tout système de capture ou de traitement du signal,
- la réalisation de biopsies quel qu'en soit la taille,
- le traitement, par exemple de tumeurs,
- l'injection locale de produits chimiques ou biologiques (cellules ou tissus transporteurs ou non de vecteurs de gènes ou des composants de ceux-ci de même que des vecteurs indépendants), produits cellulaires ou tissulaires, agents moléculaires chimiques ou physicochimiques, agents de marquage de toute sorte radioactifs ou non).

Le dispositif de l'invention peut être utilisé de façon concomitante ou successive avec des dispositifs basés sur des réactions chimiques ou biologiques autres que celles mises en oeuvre au niveau du micro-système, ou avec des dispositifs complémentaires de dilacération cellulaire, de lyse cellulaire, biopsie, d'injection ou de captures sensorielles.

Le dispositif de l'invention est remarquable en ce qu'il peut être utilisé sur tout type de substrat biologique ou chimique appartenant à des êtres vivants ou ayant vécu, humains, animaux, végétaux.

Par exemple, il est utile chez l'animal, dans les conditions normales ou dans des conditions expérimentales pour l'étude d'affection pathologique ou chez les animaux transgéniques présentant des tumeurs malignes ou différents désordres pathologiques ainsi qu'au cours de greffes de cellules, de tissus ou d'organes afin de surveiller la tolérance ou le rejet. Il permet de suivre l'évolution biologique des animaux pour la mise au point de traitements sélectifs, la sélection des races animales, la surveillance des maladies virales microbiennes, parasitaires et des maladies à agents de type prions. Le dispositif de l'invention permet d'améliorer chez l'animal les techniques de clonage reproductif ou destinées à obtenir des lignées cellulaires à activité thérapeutique régénérative issues de cellules souches embryonnaires ou de cellules souches prélevées après la naissance dites cellules souche « adultes ». Il s'agit de cellules des vaisseaux du cordon ombilical, mais également de cellules adultes autologues activées *in vitro* et présentant les caractéristiques des cellules souches capables de se différencier ultérieurement *in situ* notamment après thérapie régénérative guidée au niveau du myocarde

Chez l'être humain, le dispositif de l'invention permet de façon non-invasive ou micro-invasive de préparer l'isolement et le séquençage de gènes et/ou de l'identification de leur produit fonctionnel, de préciser un diagnostic par des méthodes chimiques ou biologiques, d'identifier avec plus de pertinence les indications thérapeutiques, de tester de nouvelles préparations biologiques ou de nouvelles molécules à activité thérapeutique, de suivre l'efficacité thérapeutique grâce à des examens orientés étagés, répétés sans risque majeur étant donné le caractère non-invasif ou micro-invasif de l'utilisation du dispositif. Le dispositif de l'invention s'applique ainsi à l'analyse de pathologie tumorale bénigne ou maligne, intéressant les tumeurs solides ou liquides comme des cancers ou des leucémies, de pathologies neurologiques, musculaires, hématologiques, cardiovasculaires, métaboliques ou dégénératives notamment qu'elles soient ou non liées à une anomalie génétique identifiée ainsi qu'à une prédisposition pathologique à composante génétique. Le dispositif de l'invention est aussi adapté à la pratique et à la surveillance de la thérapie cellulaire, de la thérapie génique, des traitements régénératifs effectués à partir de cultures de cellules souches embryonnaires, ou de cellules souches prélevées après la naissance ou encore au diagnostic préimplantatoire après fécondation *in vitro* (FIV). Le dispositif de l'invention est aussi utile sur l'être humain en développement, par exemple chez l'embryon après FIV dans le respect des règles éthiques en vigueur, chez le foetus dans le cadre du Diagnostic Prénatal en vue notamment du diagnostic d'anomalies génétiques, mais également dans le cadre de thérapeutiques prénatales menées in utero dont l'utilisation du dispositif pourrait élargir les indications. Le dispositif de l'invention trouve également une application en matière de médecine légale et au cours d'investigations propres à la police scientifique.

Chez les plantes ou dans le domaine de l'environnement, le dispositif de l'invention peut être un outil précieux pour l'identification et la sélection de variétés végétales, la réalisation d'organismes génétiquement modifiés, la surveillance de maladies virales ou parasitaires la surveillance des sols, la surveillance des déséquilibres écologiques d'ordre biologique ou chimique grâce aux applications *in vitro* et *in vivo* notamment pour l'identification des polluants biologiques ou chimiques des écosystèmes.

Dans le domaine agroalimentaire, le dispositif de l'invention est utile pour l'élaboration et le suivi des OGM, notamment pour la surveillance de la régulation et de l'expression des gènes, pour la surveillance de contaminations éventuelles au cours de l'ensemble de la chaîne alimentaire sans dénaturation préalable des denrées alimentaires, par exemple pour l'identification de microorganismes, de parasites, des virus, des rickettsies ou de protéines de type prion. Ainsi, le dispositif de l'invention est utile pour la surveillance des différentes étapes des procédés de conservation notamment par le froid.

Le dispositif de l'invention trouve également un intérêt dans le domaine paléontologique pour l'identification et l'analyse des caractéristiques chimiques ou biologiques de cellules, de tissus et d'organes momifiés ou fossilisés avec l'avantage de ne pas détruire les objets analysés, et il permet d'améliorer les outils d'identification des différentes étapes de l'évolution des espèces.

L'invention sera mieux comprise à la lecture des exemples qui suivent concernant une mise en forme particulière du dispositif de l'invention dans laquelle :
- la figure 1 représente un exemple de réalisation d'un dispositif selon l'invention.
- les figures 2 et 6 représentent un schéma du principe de l'immunocapture par technique ELISA, avec un anticorps anti-mannane et avec un anticorps anti-laminine respectivement,
- la figure 3 montre un modèle de système souple utilisé pour le guidage du support,
- la figure 4 représente un schéma du support présentant une pluralité de régions sensibilisées par un anticorps,
- la figure 5 montre un dispositif pilote dans lequel le support plastique rigide est inséré dans le système de guidage.

Ces travaux ont été réalisé avec un système de prélèvement « in situ » d'un analyte pour sa caractérisation ultérieure in vitro. Le prélèvement de l'analyte a été réalisé par immuno ou affino capture et le système de prélèvement présentait les caractéristiques suivantes :
- rigidité,
- guidage par un système souple,
- composé d'un polymère permettant le couplage de l'anticorps (Ac) ou du ligand pour une immuno ou une affino-capture de l'analyte.

Deux modèles ont été retenus pour ces travaux :
- Un premier modèle comporte l'analyse d'une candidose disséminée chez la souris par la mise en évidence des antigènes (Ag) de type mannanes de Candida albicans dans les reins ou le foie.
- Un deuxième modèle est basé sur l'analyse de la tumeur Engelbreth-Holm-Swarm (E.H.S.) chez la souris par la mise en évidence de la laminine au niveau de la tumeur de la cuisse.

Pour l'étude de ces 2 modèles, un système d'immunocapture à l'aide d'Ac anti-*Candida* ou d'Ac anti-laminine a été utilisé.

Au préalable, les différents paramètres ci-dessus ont été étudiés dans un système de microplaques :
- la concentration des Ac pour l'immunocapture
- la spécificité des Ac pour l'immunocapture
- le marquage de l'Ac révélateur par la biotine
- la détection de l'Ag biotinylé par le complexe streptavidine-enzyme

Les caractéristiques suivantes des supports ont été étudiées dans un système d'immunocapture *in vitro* :
- leur nature : polystyrène ou autre
- l'activation par différents agents
- le couplage des Ac sur les supports.

Les résultats obtenus lors de ces travaux montrent qu'il est possible (i) de fixer sur un support rigide (relié à une tige souple) deux anticorps de spécificités différentes (anti-*Candida* et anti-laminine) (ii) de prélever les analytes correspondants (antigènes *Candida* et laminine), et (iii) d'identifier ces analytes par des anticorps marqués à la biotine qui est ensuite révélée par un complexe streptavidine-enzyme.

### I - Conception et développement du système pour la mise en évidence des antigènes mannanes de C. albicans.

### 1) Principe de la réaction.

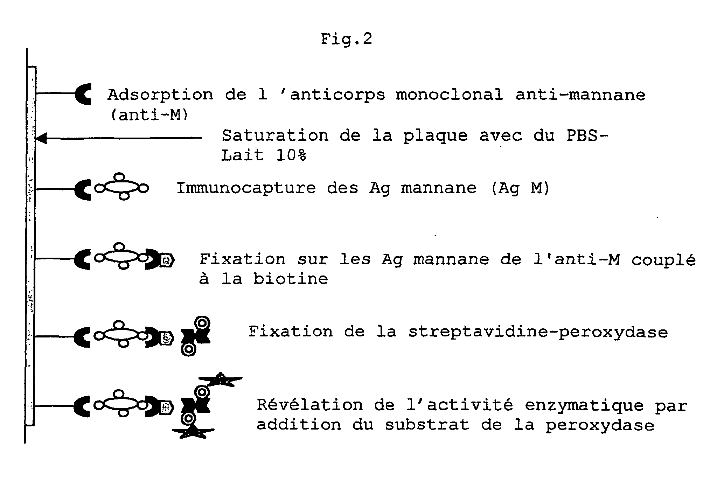

Il s'agit d'une immunocapture par technique ELISA en microplaque ou sur supports plastiques. (ELISA : Enzyme Linked Immuno Sorbent Assay) dont le principe est représenté à la figure 2 en annexe.

### 2) préparation et contrôle des réactifs.

### a) Purification de l'anticorps monoclonal anti-mannane (Ac anti-M).

L'anticorps monoclonal anti-mannane a été purifié et fourni par la SR2B.

### b) Marquage de l'anti-M par la biotine.

L'anticorps purifié, à 3 mg/ml, est dialysé une nuit à 4°C contre du tampon borate 0,1 M pH 8,8. Une solution de biotine (ester de l'acide 6-biotinamidocaproylamido-caproique et de N-hydroxysuccinimide ; Sigma®) à 10 mg/ml en DMSO est alors ajoutée à raison de 50 µg/mg d'anticorps. Après incubation de 4 h à température ambiante et sous agitation, du chlorure d'ammonium 1 M est ajouté, à raison de 20 µl/250 µg de biotine, et la solution obtenue est à nouveau incubée durant 10 min à température ambiante.

Après blocage de la réaction, l'anticorps marqué est précipité par du sulfate d'ammonium 2M et après centrifugation, le culot est repris par un tampon TBS (Tris-HCl 20 mM, pH 7.5, NaCl 150 mM) et dialysé 24 heures à +4°C contre ce même tampon.

Cet anticorps marqué est conservé sous forme d'aliquots à -20°C.

### c) Préparation de l'antigène mannane.

Les différentes étapes de préparation sont les suivantes :
- Obtention de blastospores de *Candida albicans* ATCC 66396, 48 heures sur Sabouraud Dextrose Agar à 22°C.
- Lyophilisation des levures.
- Le lyophilisat est repris par un tampon citrate pH 7.2, 0,2 M.
- Autoclavage 2 heures à 131°C (1,3 bar).
- Centrifugation de la suspension 15 minutes à 3000g.
- Ajout de 2 volumes d'éthanol absolu au surnageant et précipitation une nuit à +4°C.
- Centrifugation 15 minutes à 3000 g et lavage du culot par de l'éthanol à 60% ;
- Centrifugation 15 minutes à 3000 g et ajout d'acétone pour déshydrater le culot.
- Reprise du culot en eau distillée.
   Le dosage des hydrates de carbone est réalisé par la méthode de Dubois. La concentration en hydrates de carbone obtenue est de 21 mg/ml.

### d) Choix des supports plastiques.

### - Systèmes souples.

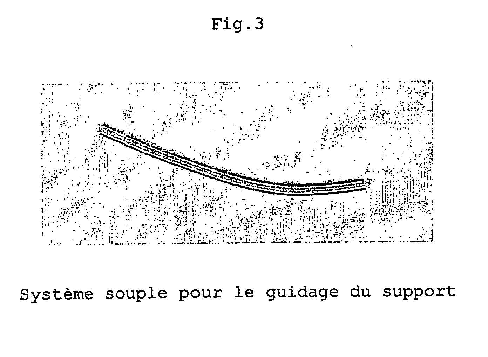

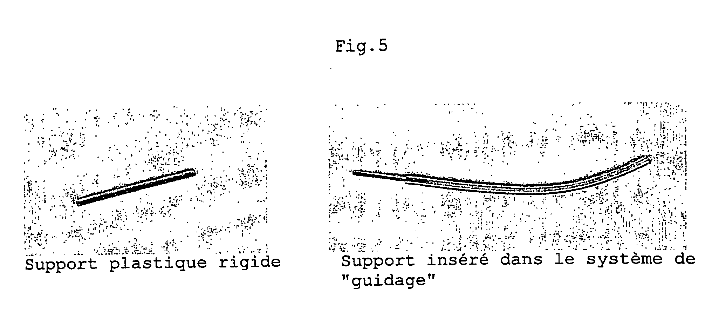

Parmi les différents systèmes souples testés pour le guidage du support, celui représenté sur les photographies données aux figures 3 et 5 en annexe à été retenu. Il s'agit d'un tube creux souple plastique dans lequel s'emboîte le support.

### - Supports plastiques rigides.

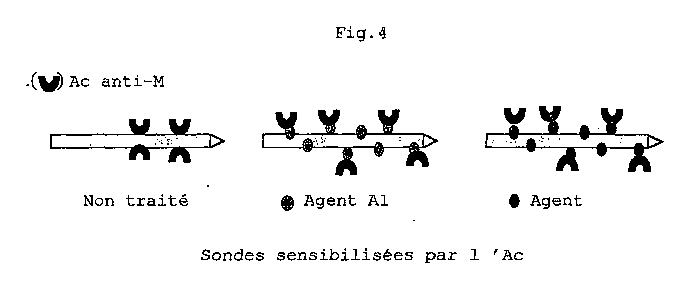

Trois types de supports plastiques ont été choisis et utilisés pour la mise au point du système, un qualifié de plastique "jaune", un qualifié de plastique "bleu" et un qualifié de plastique "blanc". Pour chacun, trois méthodes de couplage ont été testées comme représenté à la figure 4 en annexe : couplage sans traitement (Ø), couplage avec traitement par agent de couplage 1 (A1), couplage avec traitement par agent de couplage 2 (A2). Les trois types de support ont été testés non traité ou traité par A1 ou A2 et pour chaque système, les supports ont été utilisés sensibilisés par l'Ac anti-M.(U). Les supports "blanc" et "bleu" ont donné des résultats similaires. Seule le support constituée par le plastique "blanc", de longueur 2 cm, et traitée par l'agent de couplage A2 a été retenue pour la poursuite des expériences.

### e) Contrôle des réactifs et détermination des paramètres de sensibilité, spécificité en microplaque et sur supports plastiques in vitro.

### - En microplaque.

La technique a été réalisée en microplaque pour ELISA (Greiner®). Les paramètres retenus pour une sensibilité maximale sont : concentration de l'Ac anti-M pour l'immunocapture à 1 µg/ml, saturation en PBS-lait 10% 1 nuit à +4°C, concentration de l'Ac anti-M biotinylé à 0.01 mg/ml. Avec ces paramètres, la sensibilité est de 0,01 µg/ml pour la concentration en hydrates de carbone (et non pas en mannanes).

### - Sur supports plastiques.

La technique a été réalisée en microtubes à hémolyse (Fisher®). Les paramètres retenus pour une sensibilité maximale sont : concentration de l'Ac anti-M pour l'immunocapture à 10 µg/ml, saturation en PBS-lait 10% 1 nuit à +4°C, concentration de l'Ac anti-M biotinylé à 0,02 mg/ml. Avec ces paramètres, la sensibilité est de 0,01 µg/ml pour la concentration en hydrates de carbone (et non pas en mannanes).

### 3) Mise en évidence des Ag mannanes ex vivo.

### a) Détermination des paramètres pour l'obtention d'une candidose disséminée chez la souris.

- Utilisation de la souche de *Candida albicans* ATCC 66396, culture sur Sabouraud Dextrose Agar 24 hures à 37°C.
- Concentration de levures initiale 10⁷/ml en NaCl 0,15M.
- Inoculation de 100 µl par voie IV au niveau de la veine caudale à la souris.
- Obtention d'une candidose disséminée avec présence d'abcès rénaux et hépatiques.

### b) Mise en évidence de l'Ag mannanes dans le foie, les reins, le sang.

Au jour J=0 : inoculation aux souris par voie intraveineuse de blastospores de *C. albicans* ATCC 66396.

A J=2, les souris sont sacrifiées.

Le sang est recueilli par prélèvement périorbital dans des tubes de verre et après décantation, le sérum est testé pour la recherche d'Ag mannane circulant.

Les reins ou le foie sont prélevés, placés dans une boite de Pétri stérile. L'immunocapture est réalisée en enfonçant le support plastique "blanc" sensibilisé avec l'Ac anti-M dans ces organes.

Après une incubation de 15 minutes, la recherche d'Ag mannane sur les supports se fait selon le principe décrit *in vitro.*

Des reins, le foie ou le sang de souris saines constituent les témoins négatifs.

### c) Résultats.

Les résultats sont exprimés en densité optique (DO) après soustraction de la DO des témoins qui sont les souris saines.

Concernant les reins, le rein droit et le rein gauche de souris atteintes de candidoses ont été testés indépendamment par rapport à des reins de souris saine. Une différence significative est observée entre le signal obtenu pour la présence de l'Ag mannanes dans les reins "malades" et le signal obtenu pour des reins de souris saines (à titre d'exemple la différence de densité optique est d'environ 0,4).

Concernant le foie, l'Ag mannanes est également détecté par la sonde avec une différence significative (différence de densité optique 0,6).

Dans le sang (dilué au 1/10) de souris saines ou atteintes de candidoses, les Ag mannanes circulants n'ont pas été détectés.

### II - Conception et développement du système pour la mise en évidence de la laminine.

### 1) Principe de la réaction.

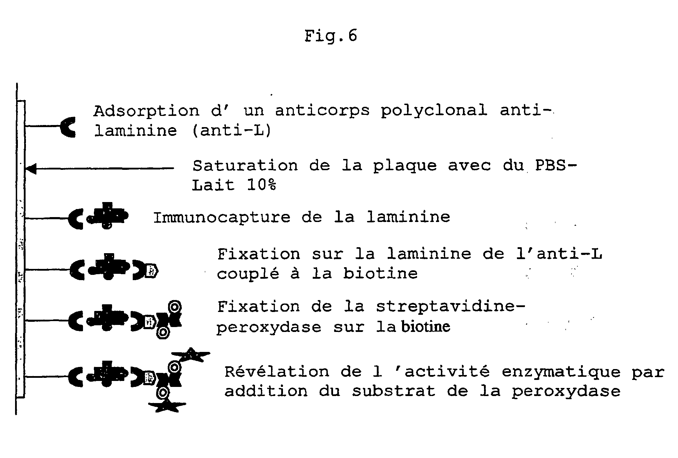

Il s'agit d'une immunocapture par technique ELISA en microplaque ou sur supports plastiques. (ELISA : Enzyme Linked Immuno Sorbent Assay) dont le principe est représenté à la figure 6 en annexe.

### 2) Préparation et contrôle des réactifs.

### a) Marquage de l'anticorps anti-laminine (Ac anti-L) par la biotine.

L'anticorps retenu pour la mise au point de la détection de la laminine est un anticorps polyclonal produit chez le lapin et purifié par affinité (Rockland®). La technique de marquage par la biotine est identique à celle utilisée pour l'anti-M.

### b) Laminine.

La laminine retenue pour la mise au point in vitro est de la laminine purifiée de souris (Sigma®).

### c) Choix des supports plastiques.

- Systèmes souples comme décrits précédemment pour *Candida albicans.*
- Supports plastiques rigides comme décrits précédemment pour *Candida albicans.*

### d) Contrôle des réactifs et détermination des paramètres de sensibilité, spécificité en microplaque et sur supports plastiques in vitro.

### - En microplaque.

La technique a été réalisée en microplaque pour ELISA (Greiner®). Les paramètres retenus pour une sensibilité maximale sont :
. Concentration de l'Ac anti-L pour l'immunocapture à 5 µg/ml.
. Saturation en PBS-lait 10%, une nuit à +4°C.
. Concentration de l'Ac anti-L biotinylé à 5 µg/ml.

Avec ces paramètres, la sensibilité est de 0,01 mg/ml pour la concentration en laminine.

### - Sur supports plastiques.

La technique a été réalisée en microtubes à hémolyse (Fisher®). Les paramètres retenus pour une sensibilité maximale sont les mêmes qu'en microplaque, à savoir :
. Concentration de l'Ac anti-L pour l'immunocapture à 5 µg/ml.
. Saturation en PBS-lait 10%, une nuit à +4°C.
. Concentration de l'Ac anti-L biotinylé à 5 µg/ml.

Avec ces paramètres, la sensibilité est de 0,01 mg/ml pour la concentration en laminine.

### 3) Mise en évidence de la laminine ex vivo.

### a) Détermination des paramètres pour l'obtention d'une tumeur "laminine" visible à l'oeil nu.

- Décongélation du sarcome E.H.S. (Engelbreth-Holm-Swarm) de souris.
- Inoculation dans les cuisses de souris.
- Après 3 semaines, les souris sont sacrifiées, les tumeurs sont prélevées, broyées et inoculées à d'autres souris.

### b) Mise en évidence de la laminine au niveau de la tumeur de la cuisse de la souris.

Les souris sont sacrifiées et l'immunocapture est réalisée en enfonçant le support plastique "blanc" sensibilisé avec l'Anti-L dans la tumeur de la cuisse. Des cuisses de souris saines constituent les témoins négatifs.

### c) Résultats.

Les résultats sont exprimés en densité optique (DO) après soustraction de la DO des témoins qui sont les souris saines.

Une différence significative est observée entre le signal obtenu pour la présence de laminine dans la tumeur et le signal obtenu pour des cuisses de souris saines (à titre d'exemple la différence de densité optique est d'environ 0,3).

### III - Couplage des deux systèmes : détection simultanée d'Ag mannanes et de laminine ex vivo.

### 1) Sensibilisation de la sonde.

Le support constitué par le plastique "blanc" et maintenu par le système de guidage souple est sensibilisé par l'Ac anti-M et l'Ac anti-L aux concentrations préalablement établies.

### 2) Mise en évidence des Ag mannanes et laminine ex-vivo.

L'immunocapture est réalisée chez :
- des souris saines (témoins)
- des souris atteintes de candidoses
- des souris avec une tumeur laminine (E.H.S.)

Des supports sont :
- soit d'abord placés dans les reins ou le foie d'une souris atteinte de candidose disséminée puis dans la tumeur (E.H.S.) de la cuisse d'une autre souris.
- soit d'abord placés dans la tumeur (E.H.S.) de la cuisse d'une souris puis dans les reins ou le foie d'une autre souris atteinte de candidose disséminée.

Les supports sont ensuite révélés par l'anti-M ou l'anti-L ou un mélange des 2.

Des témoins négatifs sont réalisés en plaçant des supports dans les reins ou le foie d'une souris saine puis dans la cuisse de la même souris (et inversement).

### 3) Résultats.

Les résultats sont exprimés en densité optique (DO) après soustraction de la DO des témoins qui sont les souris saines.

Quel que soit l'ordre dans lequel le support est implanté dans les différents organes, l'Ag mannane ou la laminine est détecté de façon significative par rapport aux souris saines quand le système est révélé par un seul des deux anticorps (DO :0,4 pour l'Ag mannane et 0.4 pour la laminine).

Quand le mélange des deux anticorps biotinylés est utilisé pour la révélation, le signal obtenu est beaucoup plus élevé (à titre d'exemple souris candidose + laminine DO=0,7).

l'Ag mannane n'a pu être détecté dans le sang des souris saines ou atteintes de candidoses disséminées.

## Revendications

1. Un dispositif pour l'analyse et/ou le traitement *in situ* dans lequel un système d'investigation et/ou de traitement coulisse dans un instrument médical, **caractérisé en ce qu'**il comprend :
(i) un micro-système d'investigation d'un substrat autre que par analyse d'un signal fluorescent, et/ou de délivrance d'agents actifs au niveau d'un substrat,
(ii) une tige souple à une extrémité de laquelle est fixé le micro-système et dont l'autre extrémité est destinée à la manoeuvre dudit micro-système,
(iii) un instrument médical possédant une lumière interne dans laquelle ladite tige souple peut coulisser,
(iv) un système de protection du micro-système amovible au niveau du substrat, et
(v) au niveau dudit micro-système, un système de dilacération de tissu ou cellule.

2. Dispositif selon la revendication 1, **caractérisé en ce que** le microsystème et la tige souple sont reliés l'un à l'autre lors de leur utilisation *in situ* et ne sont désolidarisés qu'avant ou après l'intervention in situ.

3. Dispositif selon l'une des revendications 1 ou 2, **caractérisé en ce que** le microsystème est du type comprenant un support à la surface duquel est ménagée des régions prédéfinies contenant chacune des substances chimiques ou biologiques, identiques ou différentes, d'investigation ou de traitement du substrat où est amené le micro-système grâce à la tige souple.

4. Dispositif selon l'une quelconque des revendications 1 à 3, **caractérisé en ce que** le micro-système est un micro-système récepteur/ligand mettant en oeuvre des couples de substances chimiques ou biologiques dont l'un ou l'autre ou les deux membres du couple sont fixées sur chaque région prédéfinie.

5. Dispositif selon l'une quelconque des revendications 1 à 4, **caractérisé en ce que** le microsystème est un micro-système récepteur/ligand mettant en oeuvre des couples antigènes/anticorps.

6. Dispositif selon l'une quelconque des revendications 3 à 5, **caractérisé en ce que** les substances chimiques ou biologiques sont des séquences polynucléotidique ou d'acides aminés.

7. Dispositif selon la revendication 6, **caractérisé en ce que** les substances biologiques constituent :
- un arrangement de polynucléotides capable de s'hybrider avec des acides nucléiques présents au niveau du substrat, ou
- un arrangement de peptides, polypeptides ou protéines capables de réagir avec un récepteur correspondant ou immunologiquement avec des anticorps ou des antigènes présents au niveau du substrat.

8. Dispositif selon l'une quelconque des revendications 3 à 7, **caractérisé en ce que** les substances chimiques ou biologiques sont fixées au support par des liaisons susceptibles d'être clivées in situ.

9. Dispositif selon l'une quelconque des revendications précédentes, **caractérisé en ce que** la tige souple est constituée par un instrument médical utilisé à des fins diagnostiques, thérapeutiques ou expérimentales.

10. Dispositif selon l'une quelconque des revendications précédentes, **caractérisé en ce que** le microsystème est fixé à l'une des extrémités de la tige souple par tout moyen de liaison constitué d'un système de pivot permettant l'orientation et le positionnement à partir de la tige souple dans l'espace et à distance du microsystème.

11. Dispositif selon l'une des revendications 1 à 12, **caractérisé en ce que** le micro-système est constitué d'un support se présentant sous la forme d'un ruban enroulé autour d'une extrémité de la tige souple.

12. Dispositif selon l'une quelconque des revendications précédentes, **caractérisé en ce qu'**il comprend au niveau de l'extrémité où est fixé le microsystème, un système d'aide au fonctionnement dudit microsystème, comme un système de chauffage et/ou de refroidissement ou de libération de substance biologique ou chimique.

13. Dispositif selon l'une quelconque des revendications précédentes, **caractérisé en ce qu'**il est associé à un ou plusieurs dispositifs choisis parmi un dispositif de surveillance, de réalisation de biopsie, de traitement, d'injection locale de produits biologiques ou chimique.

## Patentansprüche

1. Eine Vorrichtung zur in situ-Analyse und/oder Behandlung, in der ein System zur Untersuchung und/oder Behandlung in einem medizinischen Instrument gleitet, **dadurch gekennzeichnet, dass** sie aufweist:
i) ein Mikrosystem zur Untersuchung eines Substrats anders als durch Analyse eines fluoreszierenden Signals, und/oder zur Ausschüttung aktiver Agenzien auf Ebene eines Substrats,
ii) einen flexiblen Stab, an dessen einem Ende das Mikrosystem befestigt ist und dessen anderes Ende zur Betätigung des besagten Mikrosystems bestimmt ist,
iii) ein medizinisches Instrument mit einem internen Schlitz, in dem der flexible Stab gleiten kann,
iv) ein auf Substratebene bewegliches System zum Schutz des Mikrosystems, und
v) auf Ebene des besagten Mikrosystems ein System zur Gewebs- oder Zelldilazeration.

2. Vorrichtung nach Anspruch 1, **dadurch gekennzeichnet, dass** das Mikrosystem und der flexible Stab bei ihrer in situ-Verwendung miteinander verbunden sind und nur vor oder nach dem in situ-Eingriff voneinander getrennt sind.

3. Vorrichtung nach einem der Ansprüche 1 oder 2, **dadurch gekennzeichnet, dass** das Mikrosystem vom Typ ist, das einen Träger aufweist, auf dessen Oberfläche vordefinierte Regionen angeordnet sind, die jeweils chemische oder biologische identische oder unterschiedliche Substanzen zur Untersuchung oder Behandlung des Substrats enthalten, wohin das Mikrosystem mit dem flexiblen Stab geführt wird.

4. Vorrichtung nach einem der Ansprüche 1 bis 3, **dadurch gekennzeichnet, dass** das Mikrosystem ein Rezeptor/Ligand-Mikrosystem ist, das Paare chemischer oder biologischer Substanzen verwendet, von denen das eine oder andere oder die beiden Glieder des Paares auf jeder vordefinierten Region fixiert sind.

5. Vorrichtung nach einem der Ansprüche 1 bis 4, **dadurch gekennzeichnet, dass** das Mikrosystem ein Rezeptor/Ligand-Mikrosystem ist, dass Antigen/Antikörper-Paare verwendet.

6. Vorrichtung, nach einem der Ansprüche 3 bis 5, **dadurch gekennzeichnet, dass** die chemischen oder biologischen Substanzen Polynucleotid- oder Aminosäure-Sequenzen sind.

7. Vorrichtung nach Anspruch 6, **dadurch gekennzeichnet, dass** die biologischen Substanzen bilden:
- eine Anordnung von Polynucleotiden, die mit auf Ebene des Substrats vorhandenen Nucleinsäuren hybridisieren können, oder
- eine Anordnung von Peptiden, Polypeptiden oder Proteinen, die mit einem entsprechenden Rezeptor oder immunologisch mit auf Ebene des Substrats vorhandenen Antikörpern oder Antigenen reagieren können.

8. Vorrichtung nach einem der Ansprüche 3 bis 7, **dadurch gekennzeichnet, dass** die chemischen oder biologischen Substanzen auf dem Träger durch Verbindungen fixiert sind, die in situ gespalten sein könnten.

9. Vorrichtung nach einem der vorhergehenden Ansprüche, **dadurch gekennzeichnet, dass** der flexible Stab von einem medizinischen Instrument gebildet wird, dass bei diagnostischen, therapeutischen oder experimentellen Zwecken Verwendung findet.

10. Vorrichtung nach einem der vorhergehenden Ansprüche, **dadurch gekennzeichnet, dass** das Mikrosystem an einem der Enden des flexiblen Stabs durch jedwedes Verbindungsmittel befestigt ist, das von einem Schwenksystem gebildet wird, das die Steuerung und Positionierung mit dem flexiblen Stab im Raum und vom Mikrosystem entfernt erlaubt.

11. Vorrichtung nach einem der Ansprüche 1 bis 12, **dadurch gekennzeichnet, dass** das Mikrosystem von einem Träger gebildet wird, der sich als ein um ein Ende des flexiblen Stabs gerolltes Band darstellt.

12. Vorrichtung nach einem der vorhergehenden Ansprüche, **dadurch gekennzeichnet, dass** sie an dem Ende, wo das Mikrosystem befestigt ist, ein System zur Unterstützung der Funktion des besagten Mikrosystems aufweist, wie ein System zur Beheizung und/oder Kühlung oder Freisetzung einer biologischen oder chemischen Substanz.

13. Vorrichtung nach einem der vorhergehenden Ansprüche, **dadurch gekennzeichnet, dass** sie mit einer oder mehreren Vorrichtungen verbunden ist, die aus Vorrichtungen zur Überwachung, zur Durchführung einer Biopsie, Behandlung, lokalen Injektion von biologischen oder chemischen Produkten ausgewählt wurde/n.

## Claims

1. A device for analysis and / or *in situ* treatment in which an investigation and / or treatment system slides in a medical instrument, **characterized in that** it comprises:
(i) a micro system for investigation of a substrate other than by fluorescent signal analysis, and / or delivery of active agents to a substrate,
(ii) a flexible rod at one end of which the micro-system is fixed, the other end being. designed for manoeuvring the said micro-system,
(iii) a medical instrument with an internal hole in which the said flexible rod can slide,
(iv) a protection system for the removable micro-system at the substrate, and
(v) at the said micro-system, a system for dilaceration of the tissue or cell.

2. Device according to claim 1, **characterized in that** the micro-system and the flexible rod are connected to each other during use in situ and are only separated from each other before or after the in situ operation.

3. Device according to claim 1 or 2, **characterized in that** the micro-system is of the type comprising a support at the surface of which predefined regions are formed each containing identical or different chemical or biological substances, for investigation or treatment of the substrate to which the micro-system is brought through the flexible rod.

4. Device according to any one of claims 1 to 3, **characterized in that** the micro-system is a receptor / ligand micro-system involving pairs of chemical or biological substances, in which either one or both members of the pair are fixed onto each predefined region.

5. Device according to any one of claims 1 to 4, **characterized in that** the micro-system is a receptor / ligand micro-system using antigen / antibody pairs.

6. Device according to any one of claims 3 to 5, **characterized in that** the chemical or biological substances are polynucleotide sequences or amino acid sequences.

7. Device according to claim 6, **characterized in that** the biological substances comprise:
- an arrangement of polynucleotides capable of being hybridised with nucleic acids present in the substrate, or
- an arrangement of peptides, polypeptides or proteins capable of reacting with a corresponding receptor or reacting immunologically with antibodies or antigens present in the substrate.

8. Device according to any one of claims 3 to 7, **characterized in that** the chemical or biological substances are fixed to the support by links that could be cleaved in situ.

9. Device according to any one of the previous claims, **characterized in that** the flexible rod is composed of a medical instrument used for diagnosis, therapeutic or experimental purposes.

10. Device according to any one of the previous claims, **characterized in that** the micro-system is fixed to one of the ends of the flexible rod by any connection means consisting of a pivot system to enable remote orientation and positioning in space of the micro-system from the flexible rod.

11. Device according to any one of claims 1 to 12, **characterized in that** the micro-system is composed of a support in the form of a ribbon wound around one end of the flexible rod.

12. Device according to any one of the previous claims, **characterized in that** it comprises a system for assistance with operation of the said micro-system at the end on which the micro-system is fixed, such as a heating and / or cooling system or a system for the release of a biological or chemical substance.

13. Device according to any one of the previous claims, **characterized in that** it is used with one or several devices chosen from among a monitoring device, a device for making a biopsy, or a treatment, or local injection of biological or chemical products.
